# EUROPEAN PATENT SPECIFICATION

(11) **EP 0 543 498 B1**
(45) Date of publication and mention of the grant of the patent: **08.04.1998**
(21) Application number: 92309400.7
(22) Date of filing: 15.10.1992
(51) Int. Cl.: A61K 41/00

(54) **Injectable powder for the treatment of cancer**
Injizierbares Pulver zur Behandlung von Krebs
Poudre injectable pour la thérapie du cancer

(30) Priority: 20.11.1991 JP 329638/91; 26.02.1992 JP 72974/92
(43) Date of publication of application: 26.05.1993
(73) Proprietor: KABUSHIKI KAISHA RIKEN, Chiyoda-ku Tokyo 102 (JP); Sato, Tomoya, Fukushima-shi, Fukushima-ken (JP); Matsuki, Hidetoshi, Sendai-shi, Miyagi-ken (JP)
(72) Inventor: Minakawa, Sakae, Kabushiki Kaisha Riken, Kumagaya-shi, Saitama-ken (JP); Henmi, Hiroji, Kabushiki Kaisha Riken, Kumagaya-shi, Saitama-ken (JP)
(74) Representative: SERJEANTS

(56) References cited:
- DE-A- 3 502 998
- JP-A-23 005 750
- US-A- 5 128 147
- CHEMICAL ABSTRACTS, vol. 106, no. 10, 1986, Columbus, Ohio, US; abstract no. 72778q

## Description

### Field of the Invention

This invention relates to a an injectable powder for treating cancer. More particularly, the invention relates to a powder for cancer treatment used in a magnetic induction system employed in thermotherapy, which is one type of treatment for malignant tumors. The powder can be used both as an implant material for intra-tissue heating and in cancer chemotherapy which relies upon the release of anticancer agents.

### Description of the Prior Art

Methods of administering medicines in cancer chemotherapy usually involve administration to the entire body by injection into a vein. However, side-effects due to the toxicity of the medicines administered are a problem. The reason for this is that the toxicity values for tumorous cells and normal cells closely resemble each other in the anticancer agent irrespective of the origin and effects of the agent. Accordingly, how to make the anticancer agent concentrate selectively upon cancer cells and act for an extended period of time is one of up-to-date topics in this field.

Microcapsules and microspheres have been the subject of research as means for solving the aforesaid problems. A microcapsule consists of a capsule filled with an anticancer agent. According to the operating mechanism, the anticancer agent is released gradually into the living body through the outer wall of the capsule. Since the microcapsule itself possesses no mobility toward tumors, its effective dosage is merely in chemo-emboloid thereby which relies upon selective injection of the anticancer agent directly into the tumor-related artery. However, the release control of the anticancer agent through the microcapsule is readily determined by the physical and chemical properties as well as the structure of the material constituting the outer wall of the capsule, and the release control cannot be pursured in artificial means.

A microsphere is a particle in which an anticancer agent is dispersed in a small sphere of albumin normally used as a contrast medium in the blood circulatory system. However, the release mechanism of the medicinal substance contained is complicated and it is difficult to control release rate in artificial means since there are instances where the albumin sphere expands or dissolves in an aqueous solution.

A medicine release element which employs a magnetic material is disclosed in the specification of Japanese Utility Model No. 2-35750 as a mechanism for releasing an anticancer agent. Since the element consists of a sintered body of ferrite, a surgical operation is required in order to implant it in a living body. Furthermore, the release control of the medicinal substance is carried out by inductive heat generated in a magnetic material under the application of a magnetic field. However, since the element is a sintered body of ferrite, actual release control is considered to be difficult because the amount of anti-cancer agent generated in the sintered ferrites is totaly depending upon the loading direction of the magnetic field application.

In recent years, the combined use of chemotherapy and thermotherapy has been proven to be effective in treating cancer. There are several reasons why combined treatment promotes the effect of destroying cancerous cells. One is a compensating effect resulting from a difference between the cancer cell cycle (cell age) which is highly susceptible to heating and the cancer cell cycle which is highly susceptible to anticancer agents. Another is the prevention of a recurrence of cancer by virtue of heating after the effects of the anticancer agent diminish. Still another is that the permeability of the cancerous cell membrane to the anticancer agent is enhanced by heating.

Local thermotherapy often utilizes electromagnetic waves. Though the heating area can be highly localized if high frequency of the electromagnetic waves is employed, it is difficult to achieve heating at deeper portions by this expedient. If low frequencies are employed, it is easier to heat deeper portions but the area heated becomes too broad. Thus, local thermotherapy involves substantial problems. In addition, unless the temperature internally of the living body is measured and feedback control is applied to the electromagnetic output based upon the measured temperature, the temperature of the heated location of the body will rise excessively and this may have a harmful effect upon the body.

A method of solving these problems encountered in thermotherapy employing electromagnetic waves is being developed and referred to as "soft heating". According to this method, a thermosensitive magnetic material is implanted in a tumorous mass within a living body, and heating is applied utilizing, as a source of heat, hysteresis loss produced by exciting the magnetic material using a high-frequency magnetic field. A characterizing feature of this method is that since the treatment temperature is determined automatically by the Curie temperature of the thermosensitive element, there is no need of invasive temperature monitoring nor adjustment of the magnetic field through feed back control.

### SUMMARY OF THE INVENTION

The present invention seeks to provide a single injectable powder with which combined treatment of cancer using chemotherapy and thermotherapy can be achieved, wherein local administration of an anticancer agent can be controlled artificially and implanting of the element in a living body can be performed by injection using a catheter or indwelling needle so as not to harm the patient, and wherein intra-tissue heating is performed by soft heating without requiring adjustment of magnetic field nor invasive temperature monitoring.

Accordingly, an object of the present invention is to provide an injectable powder for treatment of cancer in soft heating using magnetic induction, in which the powder can used both for intra-tissue heating and in cancer chemotherapy which relies upon artificial release control of anticancer agents.

The present invention has been achieved as the result of a variety of research directed to solving the problems mentioned above. Specifically, according to the invention, the surface of lepidic magnetic powder having a Curie temperature of 42 to 90°C is coated with an anticancer agent, and this is further coated with a thermosensitive polymer to prepare a thermosensitive thermoelement. When a 200 KHz, 3400 A/m high-frequency alternating magnetic field is applied in physiologic saline, the injectable powder is heated to melt the thermosensitive polymeric film so that the release of the anticancer agent can be controlled artificially. After the thermosensitive polymeric film has melted to disperse the anticancer agent, additional thermotherapy is possible owing to the residual thermosensitive magnetic material. The present invention has been perfected upon confirming the foregoing effects.

In accordance with the invention, the surface of the thermosensitive magnetic powder is coated with the anticancer agent by a chemical, physicochemical or physical method. For example, the surface of the magnetic powder can be coated with the anticancer agent by mixing and stirring together a thermosensitive magnetic powder and an anticancer agent in powder form. The anticancer agent need not be applied to the entire surface of the thermosensitive magnetic powder but may be applied to the surface sporadically at a number of points. The anticancer agent can be applied upon determining the necessary amount of addition based upon the amount of thermosensitive elements injected.

It is required that the thermosensitive polymeric film melt at a temperature of 40 to 70°C. If melting takes place at a temperature less than 40°C, there is a possibility that the polymeric film will melt, even without the application of a high-frequency alternating magnetic field, when the powder is injected into a living body. If melting takes place at a temperature above 70°C, the polymeric film will not melt sufficiently when the thermosensitive magnetic powder produces heat. The desirable range of melting points is 40 to 50°C. Examples of the thermosensitive polymer which are ideal for use are wax employed in suppository, a polymer of polyacrylate and butyl methacrylate or a complex body consisting of a low melting-point polymer that is harmless to a living body.

It is required that the Curie temperature of the thermosensitive magnetic powder be greater than the melting point of the polymeric film within a range of 42 to 90°C. If the Curie temperature is less than. 42°C, warming to a therapeutic temperature effective for thermotherapy cannot be achieved. If a temperature of 90°C is exceeded, on the other hand, the region of therapeutic temperature for thermotherapy will be exceeded and overheating will occur resulting in the damage to normal tissue. The desirable range of Curie temperatures is 45 to 55°C. By way of of example, a Fe-Cr-P-C alloy such as Fe _{66.8}Cr_{13.2}P_{13.2}C_{6.8} (atomic %) or a Fe-Ni-Si-B alloy such as Fe_{17.3}Ni_{60.6}Si_{9.7}B_{12.4} can be used ideally as the magnetic powder.

As for a specific example of injecting the powder of the invention into a living body, injection can be accomplished by a catheter if the particle size diameter is made less than 150 µm.

Further, the present invention provides an injectable powder for treatment of cancer comprising a magnetic powder exhibiting a Curie temperature of 42 to 90°C, a precious metal, which is harmless to a living body, coating a surface of said magnetic powder, an anticancer agent applied to said precious metal, and a thermosensitive polymer, which melts at a temperature between 40 and 70°C, coating said precious metal and said anticancer agent.

Furthermore, the present invention provides an injectable powder for treatment of cancer comprising an oxide magnetic powder exhibiting a Curie temperature of 42 to 90°C, an electrically conductive metal film having a thickness of 0.5 to 3 µm coating all or a part of a surface of said oxide magnetic powder, an anticancer agent applied to a surface of said metal film, and a thermosensitive polymer, which melts at a temperature between 40 and 70°C, coating said metal film and said anticancer agent.

The oxide magnetic powder particles are in the shape of a disk having a diameter/thickness ratio of 3 to 10 and a diameter in a range from 30 to 100 µm, and the thickness of said electrically conductive film is 1 to 2 µm. The thermosensitive polymeric film melts at a temperature between 42 and 50°C.

### BRIEF DESCRIPTION OF THE DRAWINGS

Fig. 1 is a schematic sectional view showing a first embodiment of a powder particle according to the present invention;
Fig. 2 is a schematic sectional view showing another example of a powder particle according to the present invention;
Fig. 3 is a graph showing the temperature-elevation characteristic of the powder according to the invention when a 200 KHz, 3400 A/m high-frequency magnetic field is applied with the element immersed in physiologic saline.
Fig. 4 is a schematic sectional view showing a second embodiment of the present invention;
Fig. 5 is a schematic sectional view showing a third embodiment of the present invention; and
Fig. 6 is a modified example of the third embodiment of the present invention.

### DESCRIPTION OF THE PREFERRED EMBODIMENTS

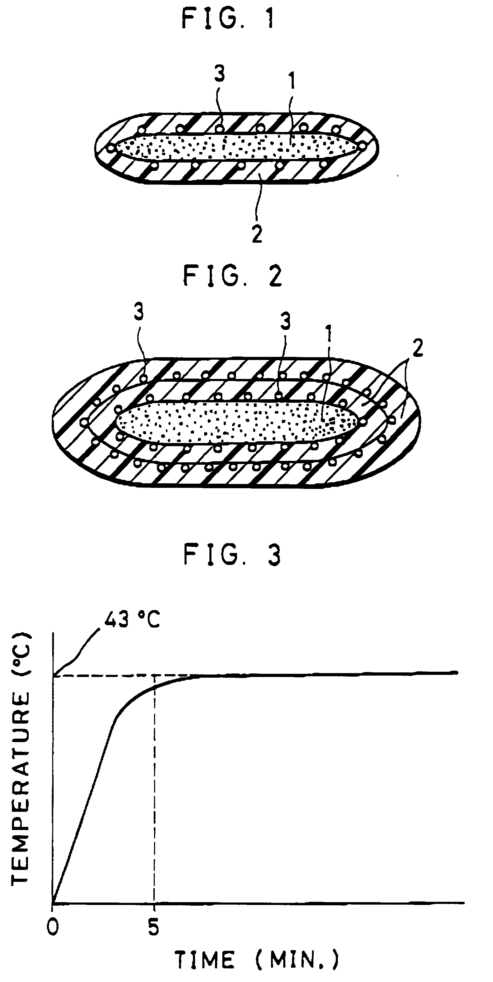

Fig. 1 is a schematic sectional view showing a particle of a powder of the invention, which is obtained by coating a thermosensitive magnetic powder having a Curie temperature of 50°C with an anticancer agent, and further coating the above with a thermosensitive polymer which melts at a temperature of 43°C. Lepidic thermosensitive magnetic powder 1 is coated with a thermosensitive polymeric film 2. Numeral 3 denotes an anticancer agent, which need not be applied to the entire surface of the magnetic powder.

In Fig. 2, which shows an another example of the invention, the thermosensitive polymeric film 2 is applied in two layers (three or more layers may be employed if desired). The layer of polymeric film near the magnetic powder 1 exhibits a high melting point, and the outer layer of the polymeric film 2 exhibits a low melting point. If three or more layers are used, the outer layers have successively lower melting points, with the outermost layer having the lowest melting point. This means that when the magnetic powder 1 produces heat, the layers of polymeric film melt selectively from the outer side so that release of the medicinal substance can be controlled by artificial application of the magnetic field. The anticancer agent 3 is embedded in both the polymeric films 1 and 2.

Fig. 3 is a graph illustrating the temperature-elevation characteristic when a high-frequency magnetic field having a frequency of 200 KHz and a field intesity of 3400 A/m is applied in physiologic saline after the polymeric film has melted out to the solvent. It will be appreciated from Fig. 3 that a therapeutic temperature for thermotherapy is attained within five minutes.

By using an injectable powder for cancer treatment in accordance with the present invention, release of the anticancer agent into cancerous cells can be controlled artificially and effectively. Furthermore, it is possible to perform thermotherapy by auto-temperature control owing to the magnetic powder which remains after release of the anticancer agent.

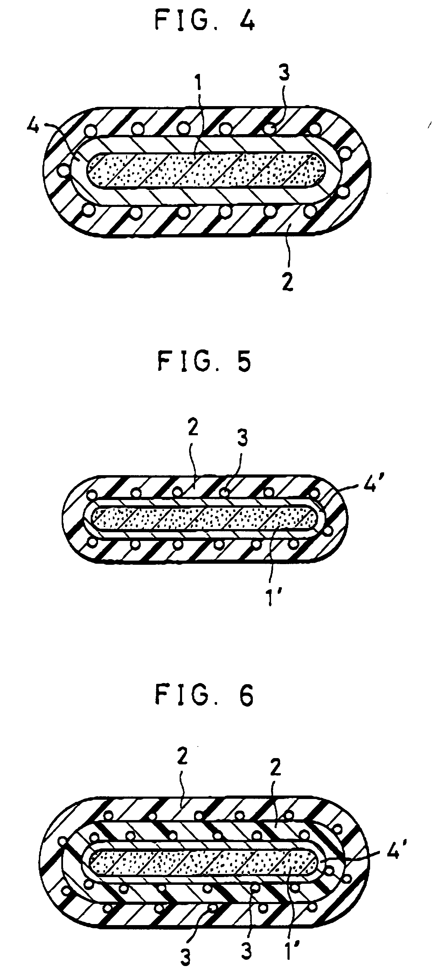

In Fig. 4, the magnetic powder 1 is coated with a precious or noble metal 4 which is harmless to a living body. The magnetic material 1, the polymer film 2 and the anticancer agent 3 are the same materials as used in Fig. 1.

In Figs. 5 and 6, an oxide magnetic material 1' exhibiting Curie temperature of 42 to 90°C is used as core material in place of the magnetic material 1 and an electrically conductive metal film 4' having a thickness of 0.5 to 3 µm is used to coat all or a part of a surface of the oxide magnetic material 1'. The film 2 and the agent 3 are the same materials as used in Figs. 1 and 4. The oxide magnetic material 1' is in the shape of a disk having a diameter/thickness ratio of 3 to 10 and a diameter in a range of 30 to 100 µm, and the thickness of said electrically conductive film 4' is 1 to 2 µm.

In Fig. 6, which shows a modification of Fig. 5, the thermosensitive polymeric film 2 is applied in two layers (three or more layers may be employed if desired). The layer of polymeric film 2 near the oxide magnetic material 1' exhibits a high melting point, and the outer layer of the polymeric film 2 exhibits a low melting point. If three or more layers are used, the outer layers have successively lower melting points, with the outermost layer having the lowest melting point. This means that when the oxide magnetic material 1' generates heat, the layers of polymeric film 2 melt selectively from the outer side so that release of the medicinal substance can be controlled by artificial application of the magnetic field.

## Claims

1. An injectable powder for the treatment of cancer, comprising:
a magnetic powder (1) exhibiting a Curie temperature of 42°C to 90°C;
an anticancer agent (3) applied to a surface of the magnetic powder (1); and
a thermosensitive polymer (2), which melts at a temperature of 40°C to 70°C and below the Curie temperature of the magnetic powder (1), coating both the magnetic powder (1) and the anticancer agent (3).

2. An injectable powder for the treatment of cancer, comprising:
a magnetic powder (1) exhibiting a Curie temperature of 42°C to 90°C;
a precious metal (4), which is harmless to a living body, coating a surface of the magnetic powder (1);
an anticancer agent (3) applied to the precious metal (4); and
a thermosensitive polymer (2), which melts at a temperature of 40°C to 70°C and below the Curie temperature of the magnetic powder (1), coating both the precious metal (4) and the anticancer agent (3).

3. An injectable powder for the treatment of cancer, comprising:
an oxide magnetic powder (1'), exhibiting a Curie temperature of 42°C to 90°C;
an electrically conductive metal film (4') having a thickness of 0.5 to 3 µm coating all or a part of a surface of the oxide magnetic powder (1');
an anticancer agent (3) applied to a surface of the metal film (4'); and
a thermosensitive polymer (2), which melts at a temperature of 40°C to 70°C and below the Curie temperature of the oxide magnetic powder (1'), coating both the metal film (4') and the anticancer agent (3).

4. A powder according to claim 3, wherein the particles of the oxide magnetic powder (1') are in the shape of a disk having a diameter/thickness ratio of 3 to 10 and a diameter in the range of 30 to 100 µm.

5. A powder according to claim 3 or claim 4, wherein the thickness of the electrically conductive film is 1 to 2 µm.

6. A powder according to any of claims 3 to 5, wherein the thermosensitive polymer (2) melts at a temperature of 42°C to 50°C.

7. A powder according to any preceding claim, wherein the Curie temperature of the magnetic material (1 or 1') is from 45°C to 55°C.

## Patentansprüche

1. Injizierbares Pulver zur Krebsbehandlung, mit einem magnetischen Pulver (1), das eine Curie-Temperatur von 42 °C bis 90 °C aufweist, mit einem Antikrebsagens (3), das auf die Oberfläche des magnetischen Pulvers (1) aufgebracht ist, und mit einem wärmeempfindlichen Polymer (2), das bei einer Temperatur von 40 °C bis 70 °C und unterhalb der Curie-Temperatur des magnetischen Pulvers (1) schmilzt, das das magnetische Pulver (1) und auch das Antikrebsagens (3) ummantelt.

2. Injizierbares Pulver zur Krebsbehandlung, mit einem magnetischen Pulver (1), das eine Curie-Temperatur von 42 °C bis 90 °C aufweist, mit einem Edelmetall (4), das für ein Lebewesen harmlos ist, das eine Oberfläche des magnetischen Pulvers (1) ummantelt, mit einem Antikrebsagens (3), das auf das Edelmetall (4) aufgebracht ist, und mit einem wärmeempfindlichen Polymer (2), das bei einer Temperatur von 40 °C bis 70 °C und unterhalb der Curie-Temperatur des magnetischen Pulvers (1) schmilzt, das das Edelmetall (4) und auch das Antikrebsagens (3) ummantelt.

3. Injizierbares Pulver zur Krebsbehandlung, mit einem magnetischen Oxidpulver (1'), das eine Curie-Temperatur von 42 °C bis 90 °C aufweist, mit einem elektrisch leitfähigen Metallfilm (4') mit einer Dicke von 0,5 bis 3 µm, der die gesamte oder einen Teil der Oberfläche des magnetischen Oxidpulvers (1') ummantelt, mit einem Antikrebsagens (3), das auf einer Oberfläche des Metallfilms (4') aufgebracht ist, und mit einem wärmeempfindlichen Polymer (2), das bei einer Temperatur von 40 °C bis 70 °C und unterhalb der Curie-Temperatur des magnetischen Oxidpulvers (1') schmilzt, das den Metallfilm (4') und auch das Antikrebsagens (3) ummantelt.

4. Pulver nach Anspruch 3, bei dem Teilchen des magnetischen Oxidpulvers (1') die Form einer Scheibe mit einem Durchmesser-/Dickenverhältnis von 3 bis 10 und einem Durchmesser im Bereich von 30 bis 100 µm haben.

5. Pulver nach Anspruch 3 oder Anspruch 4, bei dem die Dicke des elektrisch leitfähigen Films 1 bis 2 µm beträgt.

6. Pulver nach einem der Ansprüche 3 bis 5, bei dem das wärmeempfindliche Polymer (2) bei einer Temperatur von 42 °C bis 50 °C schmilzt.

7. Pulver nach einem der vorhergehenden Ansprüche, bei dem die Curie-Temperatur des magnetischen Materials (1) oder (1') zwischen 45 °C und 55 °C liegt.

## Revendications

1. Poudre injectable pour le traitement du cancer, comprenant :
une poudre magnétique (1) présentant un point de Curie de 42°C à 90°C ;
un agent anticancéreux (3) appliqué sur la surface de la poudre magnétique (1) ; et
un polymère thermosensible (2), qui fond à une température de 40°C à 70°C et au-dessous du point de Curie de la poudre magnétique (1), recouvrant à la fois la poudre magnétique (1) et l'agent anticancéreux (3).

2. Poudre injectable pour le traitement du cancer, comprenant :
une poudre magnétique (1) présentant un point de Curie de 42°C à 90°C ;
un métal précieux (4), qui est inoffensif pour un corps vivant, recouvrant la surface de la poudre magnétique (1) ;
un agent anticancéreux (3) appliqué sur le métal précieux (4) ; et
un polymère thermosensible (2), qui fond à une température de 40°C à 70°C et au-dessous du point de Curie de la poudre magnétique (1), recouvrant à la fois le métal précieux (4) et l'agent anticancéreux (3).

3. Poudre injectable pour le traitement du cancer, comprenant :
une poudre magnétique à base d' oxyde (1'), présentant un point de Curie de 42°C à 90°C ;
un film de métal électroconducteur (4') ayant une épaisseur de 0,5 à 3 µm, recouvrant la totalité ou une partie de la surface de la poudre magnétique à base d'oxyde (1') ;
un agent anticancéreux (3) appliqué sur la surface du film métallique (4') ; et
un polymère thermosensible (2), qui fond à une température de 40°C à 70°C et au-dessous du point de Curie de la poudre magnétique à base d'oxyde (1'), recouvrant à la fois le film métallique (4') et l'agent anticancéreux (3).

4. Poudre selon la revendication 3, dans laquelle les particules de la poudre magnétique à base d'oxyde (1') ont la forme d'un disque ayant un rapport de diamètre/épaisseur de 3 à 10 et un diamètre compris entre 30 et 100 µm.

5. Poudre selon la revendication 3 ou la revendication 4, dans laquelle l'épaisseur du film électroconducteur est de 1 à 2 µm.

6. Poudre selon l'une quelconque des revendications 3 à 5, dans laquelle le polymère thermosensible (2) fond à une température de 42°C à 50°C.

7. Poudre selon l'une quelconque des revendications précédentes, dans laquelle le point de Curie du matériau magnétique (1 ou 1') est de 45°C à 55°C.
